# EUROPÄISCHE PATENTANMELDUNG

(11) **EP 4 516 250 A1**
(43) Veröffentlichungstag der Anmeldung: **05.03.2025**
(21) Anmeldenummer: 23194513.0
(22) Anmeldetag: 31.08.2023
(51) Int. Cl.: A61B 18/12, A61B 18/14

(54) **BEHANDLUNGSEINRICHTUNG UND STEUERVERFAHREN**

(71) Anmelder: Erbe Elektromedizin GmbH, 72072 Tübingen (DE)
(72) Erfinder: Ripplinger, Thomas, 72072 Tübingen (DE)
(74) Vertreter: Rüger Abel Patentanwälte PartGmbB

(57) **Zusammenfassung**

Die erfindungsgemäße Behandlungseinrichtung (10) umfasst ein an ein speisendes Gerät (12) angeschlossenes Instrument (11). Das Instrument (11) und das Gerät (12) können sich gegenseitig steuern. Mittels einer an dem Instrument (11) oder anderweitig vorgesehenen Aktivierungseinrichtung (24) ist der Generator in einer ersten oder in einer zweiten Betriebsart aktivierbar. Das Instrument (11) enthält eine Weiche (30), um die von dem Generator gelieferte elektrische Leistung auf ein erstes oder ein zweites Elektrodenpaar (20/21) oder (22/21) zu leiten. Die Weiche (30) wird von dem Generator (12) gesteuert. Dies eröffnet die Möglichkeit Behandlungsabläufe in dem Generator zu speichern und mit dem Instrument (11) auszuführen, ohne dass der Nutzer Betriebsarten umschalten müsste.

## Beschreibung

Die Erfindung betrifft eine Behandlungseinrichtung, die insbesondere zur Koagulation und Dissektion von biologischem Gewebe eines menschlichen oder tierischen Patienten geeignet ist.

Behandlungseinrichtungen zur Gewebekoagulation und/oder Dissektion sind in verschiedenen Ausführungsformen bekannt.

Die EP 3 895 642 A1 beschreibt eine solche aus Generator und Instrument bestehende Behandlungseinrichtung, bei der der Generator dazu eingerichtet ist, eine zum Koagulieren von biologischem Gewebe ausreichende elektrische Spannung zu erzeugen. In dem Instrument ist ein Transformator vorgesehen, der an eine Schneidelektrode angeschlossen und dazu eingerichtet ist, aus der zur Koagulation eingerichteten Spannung durch Transformation eine zum Schneiden von Gewebe ausreichende Spannung zu erzeugen. Zur Aktivierung der Elektrodenanordnung dient ein elektronischer Schalter, der über einen am Instrument vorgesehenen Taster aktvierbar ist. Mit diesem oder einem alternativen zweiten Taster, wird jeweils ein Aktivierungssignal für den Generator erzeugt, um diesen ein- und auszuschalten.

Die EP 1 287 788 A1 ist eine Behandlungseinrichtung mit einem Generator und einem Instrument bekannt, wobei das Instrument einen Schalter enthält, um alternativ ein erstes oder ein zweites Elektrodenpaar mit dem Generator zu verbinden. Die Steuerung der Aktivierung des Generators und Instruments erfolgt über eine Aktivierungseinrichtung in Gestalt eines Hand- oder Fußschalters. Entsprechend wird der Leistungsfaktor des Generators umgeschaltet.

Weitere Behandlungseinrichtungen sind aus der EP 1 545 360 A1, der US 2010/0137854 A1, der EP 1 599 146 B1 und der EP 1 711 116 B1 bekannt.

Es ist Aufgabe der Erfindung, eine verbesserte Behandlungseinrichtung anzugeben.

Die erfindungsgemäße Behandlungseinrichtung umfasst einen Generator und ein von diesem gespeistes Instrument. Der Generator ist dazu eingerichtet, in Abhängigkeit von einem Generatorsteuersignal eine elektrische Spannung abzugeben, die für einen ersten Behandlungsmodus, beispielsweise die Gewebekoagulation, oder alternativ für einen zweiten Behandlungsmodus, beispielsweise die Gewebedissektion, geeignet ist. Der Generator ist zwischen den beiden genannten Behandlungsmodi umschaltbar ausgebildet. Bedarfsweise kann der Generator auch zwischen anderen und/oder mehreren Behandlungsmodi umschaltbar sein.

Der Generator ist dazu eingerichtet, dazu von einem Generatorsteuersignal gesteuert zu werden, das den Behandlungsmodus vorgibt. Weiter ist der Generator dazu eingerichtet ein Weichensteuersignal abzugeben, das eine vorzugsweise in dem Instrument angeordnete Weiche steuert, um die von dem Generator abgegebene Spannung und somit auch den von dem Generator gelieferten Strom an die jeweils für den gewählten Behandlungsmodus geeignete Elektrode (bzw. das betreffende Elektrodenpaar) zu leiten.

Bei der Erfindung wird zur Übermittlung des Generatorsteuersignals, sowie auch zur Übermittlung des Weichensteuersignals ein Signalträger genutzt. Der Generator ist dazu eingerichtet, den Signalträger bereitzustellen. Das Instrument ist über eine Leitung mit dem Generator verbunden und an den Signalträger angeschlossen, so dass der Signalträger von dem Generator an das Instrument übermittelt wird. Das Signalträger ist vorzugsweise eine zum Beispiel rechteckförmige Wechselspannung im Tonfrequenzbereich, zwischen 20 Hz und 20 kHz. Vorzugsweise beträgt die Frequenz des Signalträgers etwa 2 kHz. Es kann sich bei dem Signalträger um ein Wechselstromsignal handeln, das mit der angegebenen Frequenz beispielsweise zwischen einem positiven und einem negativen Wert alterniert. Der positive Wert und der negative Wert können den gleichen Betrag haben.

Dem Instrument kann eine Aktivierungseinrichtung zugeordnet sein, die dazu eingerichtet ist, das Generatorsteuersignal zu erzeugen. Dazu kann die Aktivierungseinrichtung dazu eingerichtet sein, für verschiedene gewünschte Betriebsmodi unterschiedliche Widerstände mit diesem Wechselstromsignal zu beaufschlagen. Die sich ergebende Spannung ist dann das Generatorsteuersignal, das von einer Steuereinrichtung des Generators erfasst und zur Steuerung desselben herangezogen wird.

Alternativ kann es sich bei dem Signalträger auch um ein Wechselspannungssignal mit einer geeigneten Frequenz, beispielsweise 20 Hz bis 20 kHz, beispielsweise 2 kHz handeln, wobei in dem Instrument oder in der Aktivierungseinrichtung wiederum verschiedene, über Taster aktivierbare Widerstände mit dem Signalträger beaufschlagbar sind. In diesem Fall ist der sich einstellende Wechselstrom das Generatorsteuersignal, wobei die Steuereinrichtung des Generators dazu eingerichtet ist, den Generator entsprechend zu steuern.

Die Aktivierungseinrichtung kannn in dem Instrument oder von diesem gesondert in einem eigenen Bedienteil angeordnet sein.

Erfindungsgemäß enthält das Instrument der Behandlungseinrichtung eine Weichenschaltung, die entweder die für den ersten Behandlungsmodus oder die für den zweiten Behandlungsmodus vorgesehene Elektrode mit der von dem Generator herkommenden Leitung verbindet. Dabei kann für beide Elektroden ein und dieselbe Gegenelektrode vorgesehen sein, die mit der jeweiligen Elektrode des ausgewählten Behandlungsmodus ein Elektrodenpaar bildet. Die Weiche kann auch darauf eingerichtet sein, eine Elektrode des ersten Behandlungsmodus für den zweiten Behandlungsmodus als Neutralelektrode zu schalten.

Die Weichenschaltung wird durch ein Weichensteuersignal entsprechend dem gewählten Behandlungsmodus gesteuert. Der Generator ist dazu eingerichtet, das Weichensteuersignal zu erzeugen und dem Signalträger aufzuprägen. Beispielsweise kann der Signalträger ein variables Tastverhältnis aufweisen, so dass der Mittelwert des zwischen einem positiven und einem negativen Wert alternierenden Signalträgers entweder positiv oder negativ wird. Dabei kann ein erster Mittelwert dem ersten Behandlungsmodus und ein zweiter Mittelwert dem zweiten Behandlungsmodus zugeordnet sein. Die Weichenschaltung kann dazu eingerichtet sein, über die Erfassung der verschiedenen Mittelwerte des Signalträgers entsprechend die erste oder die zweite Elektrode mit der Leitung und somit mit dem Generator zu verbinden. Der Mittelwert des Signalträgers ist dann das Weichensteuersignal.

Das erfindungsgemäße System gestattet die Steuerung der in dem Instrument vorgesehenen Weiche durch den Generator. Der Generator wird wiederum durch die Aktivierungseinrichtung gesteuert, die in dem Instrument oder einem gesonderten Element, beispielsweise einem Pedal, angeordnet sein kann.

Bei der Erfindung wird der Behandlungsstrom kontrolliert nur der für die jeweilige Behandlung vorgesehenen Elektrode zugeführt. Die in dem Instrument vorgesehene Weiche ist dabei über das den Generator enthaltende Gerät gesteuert. Die Weiche ist dazu eingerichtet nur diejenige(n) Elektrode(n) mit dem Generator zu verbinden, die zu dem ausgewählten Mode gehören, in dem das Gerät arbeitet.

Weil durch die von dem Generator gesteuerte Weiche lediglich die aktivierte Elektrode Strom führt und kein Strom über die einem anderen Mode zugeordnete Elektrode fließt, lassen sich die Generatorspannung und/oder der Generatorstrom besonders genau erfassen und feinfühlig regeln. Über die von dem Generator abgegebene Spannung und den Strom sowie die Phasenverschiebung zwischen beiden kann die abgegebene Leistung ermittelt werden. Außerdem lässt sich der Gewebewiderstand errechnen und wunschgemäß regulieren.

Durch schnelles alternierendes Umschalten der Weiche und eine entsprechende Führung der Generatorspannung kann eine Aktivierung nachgebildet werden, bei der gleichzeitig beide Elektroden bestromt werden. So ist zum Beispiel bei einem Koagulations- und Dissektionsinstrument eine Vorversiegelung eines Gefäßes oder anderen Gewebes möglich, bevor der Gewebeschnitt im Rahmen einer Dissektion stattfindet. Werden Arterien oder andere Gefäße mit einem zangenartigen Instrument gefasst, das auch eine Schneidelektrode enthält, können zunächst die Koagulationselektroden bestromt werden und der Schnitt verzögert oder verlangsamt durchgeführt werden. Dies erhöht die Behandlungssicherheit, indem ein Herausrutschen von noch nicht gänzlich versiegelten Arterien aus dem geschlossenen zangenartigen Instrument vermieden wird.

Ein weiterer Vorzug der erfindungsgemäßen Behandlungseinrichtung liegt darin, dass in dem Instrument kein Transformator erforderlich ist, wodurch das Instrument besonders leicht und schlank und mit geringem technischem Aufwand bereitgestellt werden kann.

Der Generator ist vorzugsweise ein Hochfrequenzgenerator der darauf eingerichtet ist, eine Wechselspannung mit einer Frequenz zwischen 100 kHz und 5 MHz abzugeben. Vorzugsweise liegt die Generatorfrequenz zwischen 300 kHz und 600 kHz und ist sowohl in ihrer Spitzen-Spitzen-Spannung, als auch zum Beispiel in verschiedenen Strom und/oder Leistungsbegrenzungen variierbar. Außerdem kann der Generator darauf eingerichtet sein, eine modulierte, beispielsweise ein/aus-getastete Spannung bereitzustellen, um verschiedene gewünschte Crestfaktoren zu erzielen.

Die Erfindung eignet sich insbesondere für zangenartige Instrumente mit feststehender Schneidelektrode, wobei der erste Behandlungsmodus ein Koagulationsmodus sein kann. Zur Koagulation sind in dem zangenartigen Maulteil an den beiden Branchen Elektroden vorgesehen. Eine der beiden Elektroden kann als Neutralelektrode betrachtet werden, während die andere Elektrode der anderen Branche die zugeordnete Koagulationselektrode ist. Die Neutralelektrode und die Koagulationselektrode bilden das Elektrodenpaar für die Gewebekoagulation und/oder Gewebefusion.

Der zweite Behandlungsmodus kann ein Dissektionsmodus sein. In diesem wird die feststehende Schneidelektrode aktiviert. Wiederum kann eine der beiden an den Branchen vorgesehenen Elektroden die Neutralelektrode bilden. Es ist auch möglich, die Weichenschaltung so auszubilden, dass sie im Dissektioonsmodus die beiden an den Branchen vorgesehenen Elektroden elektrisch miteinander verbindet. Die beiden an den Branchen vorgesehenen Elektroden sind dann in diesm Mode die neutralen Elektroden.

Grundsätzlich ist die Erfindung auch für andere Instrumente geeignet, beispielsweise Instrumente mit Dissektions- und Ablationsfunktion oder dergleichen.

Mit dem erfindungsgemäßen Konzept lässt sich sicherstellen, dass das Generatorsteuersignal zeitlich nach dem Weichensteuersignal beginnend und zeitlich vor dem Weichensteuersignal endend festgelegt ist. Dadurch kann sichergestellt werden, dass der Generator dann und nur dann eine für einen Mode vorgesehene Spannung abgibt, wenn und solange die zugeordnete Elektrode mit dem Generator verbunden ist.

Weiter ermöglicht das erfindungsgemäße Konzept dank der Steuerung der im Instrument vorgesehenen Weiche durch den Generator die Durchführung automatischer Mode-Abläufe, bei denen einem vorgesehenen Programm folgend nacheinander verschiedene Modi zur Anwendung kommen. Beispielsweise kann bei einem Versieglungs- und Dissektionsinstrument sichergestellt werden, dass zunächst die Gefäß- oder Gewebeversiegelung begonnen und erst danach die Dissektion (der Gewebeschnitt) durchgeführt wird. In diesem Fall genügt es, wenn die Aktivierungseinrichtung als Generatorsteuersignal ein einfaches Ein/Aus-Signal abgibt. Der Modablauf kann dann von dem Generator selbst gesteuert werden, der auch die Steuerung der Instrumentenweiche übernimmt.

Weitere Einzelheiten vorteilhafter Ausführungsformen der Erfindung sind Gegenstand von Ansprüchen sowie der Zeichnung und der dazugehörigen Beschreibung. In der Zeichnung zeigen:
Figur 1 eine beispielhafte Darstellung eines Instruments und eines zugeordneten Generators in schematisierter Übersichtsdarstellung,
Figur 2 die erfindungsgemäße Behandlungseinrichtung mit Generator und Instrument in Blockdarstellung,
Figur 3 das zu der erfindungsgemäßen Behandlungseinrichtung gehörige Instrument in Blockdarstellung,
Figur 4 einen in dem Instrument nach Figur 3 verwendeten elektronischen Schalter,
Figur 5 den Signalträger als Zeitdiagramm,
Figur 6 das Weichensteuersignal im zeitlichen Verlauf als schematisches Diagramm,
Figur 7 die von dem Generator abgebebene hochfrequente Spannung als schematisches Diagramm und
Figur 8 das aus dem Tastverhältnis des Signalträgers nach Figur 5 hergeleitete Weichensteuersignal als Diagramm.

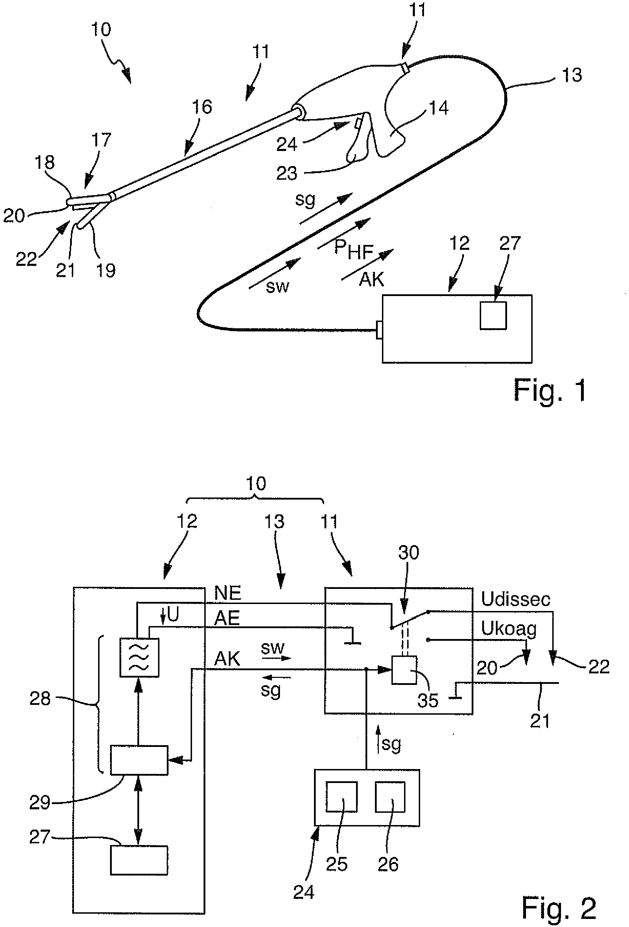

In Figur 1 ist eine Behandlungseinrichtung 10 veranschaulicht, zu der ein Instrument 11 sowie ein Gerät 12 gehören, an welches das Instrument 11 über ein Kabel 13 angeschlossen ist. Das Instrument 11 ist in Figur 1 beispielhaft als laparoskopisches Instrument veranschaulicht. Es weist dazu ein mit einem Griff 14 versehenes Gehäuse 15 auf, von dem sich ein Schaft 16 in distaler Richtung erstreckt, an dessen Ende ein zangenartiges Werkzeug 17 gehalten ist. Dieses Werkzeug 17 dient der Gewebekoagulation und Gewebedissektion, also beispielsweise der Versiegelung von Gefäßen, zum Beispiel Blutgefäßen, und der Trennung derselben.

Das Werkzeug 17 weist an seinen Branchen 18, 19 innen Koagulationselektroden 20, 21 auf, zwischen denen Gewebe gefasst und zusammengedrückt gehalten werden kann. Durch Stromfluß zwischen den Koagulationselektroden 20, 21 wird das Gewebe erhitzt und koaguliert. Z.B. können damit auch Blutgefäße oder andere Hohlstrukturen fusioniert und damit versiegelt werden.

Wenigstens eine der Branchen 18, 19 ist zusätzlich mit einer Schneidelektrode 22 versehen. Diese Schneidelektrode dient dazu, das Gewebe zu durchtrennen. Sie arbeitet typischerweise mit einer höheren Spannung als die Koagulationselektroden und mit höherer lokaler Stromkonzentration.

Das Instrument 11 ist in dieser Form lediglich zur beispielhaften Veranschaulichung angegeben. Es kann auch als Instrument für den offenchirurgischen Einsatz oder für den endoskopischen Einsatz ausgebildet sein. Anstelle der Koagulationselektroden 20, 21 und der Schneidelektrode 22 können auch für andere Behandlungsaufgaben vorgesehene Elektroden, zum Beispiel Ablationselektroden oder dergleichen vorgesehen sein, die mittels einer Umschalteinrichtung zu unterschiedlichen Zeitpunkten aktiviert und dazu mit dem Generator verbunden werden.

Zum Öffnen und Schließen der Branchen 18, 19 ist an dem Gehäuse 14 mindestens ein Betätigungselement 23, beispielsweise in Gestalt eines Handhebels angeordnet. Außerdem können zum Aktivieren der Elektroden 20, 21, 22 eine Aktivierungseinrichtung 24 mit einem oder mehreren Schaltern oder Tastern 25, 26 vorgesehen sein (siehe Figur 2). Die Aktivierungseinrichtung 24 kann, wie Figur 1 veranschaulicht, Teil des Instruments 11 sein. Alternativ kann die Aktivierungseinrichtung 24 auch in einem gesonderten Gehäuse angeordnet und zum Beispiel als Hand- oder Fußschalter ausgebildet sein.

Außerdem kann das Gerät 12 eine Bedieneinheit 27 aufweisen, über die der Betrieb des Instruments 11 beinflussbar ist. Die Bedieneinheit 27 kann dazu eingerichtet sein, die Betriebsparameter für die Behandlungsmodi coag, cut einzustellen oder andere Einstellungen vorzunehmen.

Die Behandlungseinrichtung 10 ist in Figur 2 gesondert und in abstrakter Form veranschaulicht. Das Gerät 12 enthält einen Generator 28, der dazu eingerichtet ist, eine hochfrequente Spannung U abzugeben, die zur Durchführung verschiedener Behandlungen an biologischem Gewebe geeignet ist. Die Spannung U ist vorzugsweise eine hochfrequente Spannung, deren Spitzenwert im Bereich von etwa 100 V bis zu mehreren 100 V oder auch höher variierbar ist. Außerdem ist der Generator 28 vorzugsweise dazu eingerichtet, diese hochfrequente Spannung U zu modulieren. Beispielsweise kann die Spannung U als kontinuierliche Spannung oder auch als ein/aus-getastete Spannung abgegeben werden, wobei das Puls/Pause-Verhältnis entsprechend dem gewünschten Mode der Behandlung variierbar sein kann. Beispielsweise kann der Generator 28 darauf eingerichtet sein, die Spannung Ucoag für einen ersten Behandlungsmodus coag, beispielsweise einen Koagulationsmodus, abzugeben, bei dem die Spannung zwischen den beiden Elektroden 20, 21 wirksam werden soll. Außerdem kann der Generator 28 darauf eingerichtet sein, für einen zweiten Behandlungsmodus cut, beispielsweise einen Dissektionsmodus, eine Spannung Ucut abzugeben, die zum Gewebeschnitt ausreichend ist und zwischen den Elektroden 22, 21 wirksam werden soll.

Die Steuerung des Generators erfolgt entsprechend einem Generatorsteuersignal sg. Es kann eine Steuereinrichtung 29 vorgesehen sein, die dazu eingerichtet ist, das Generatorsteuersignal sg auszuwerten und zur Steuerung des Generators 28 heranzuziehen. Die Steuereinrichtung 29 kann Teil des Geräts 12 und auch mit der Bedieneinheit 27 verbunden sein.

Das Kabel 13 enthält mindestens drei Leitungen NE, AE und AK, die zu dem Instrument 11 führen. Während die Leitung AE Neutralpotenzial führt, gibt der Generator 28 über die Leitung NE die Behandlungsspannung und den Behandlungsstrom an das Instrument 11 ab.

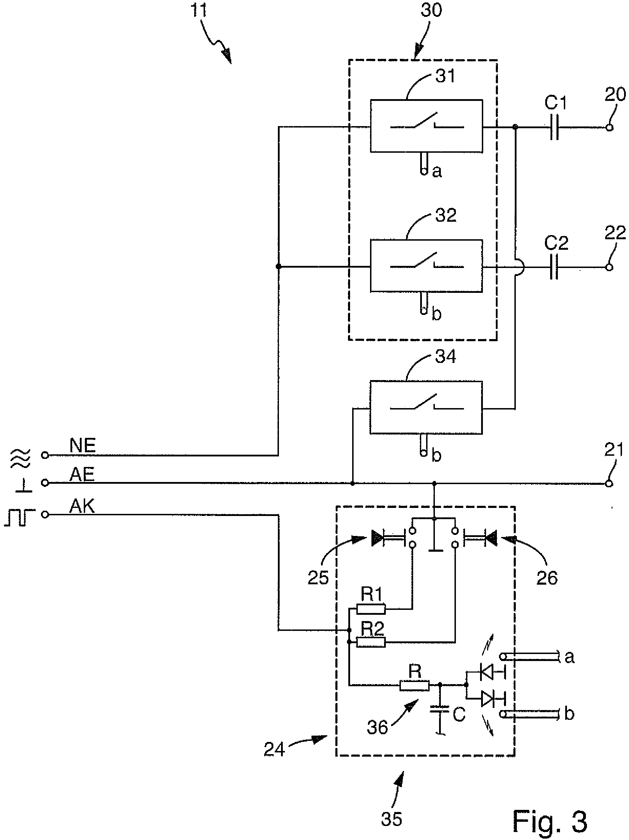

Das Instrument 11 enthält eine elektrische Weiche 30, mittels derer die von der Leitung NE herkommende Behandlungsspannung und entsprechend der Behandlungsstrom wahlweise auf die zum Koagulieren vorgesehene Elektrode 20 oder die zum Schneiden vorgesehene Elektrode 22 geleitet wird. Die Weiche 30 ist insoweit eine Umschalteinrichtung, die über ein Weichensteuersignal sw von der zu dem Generator 28 gehörigen Steuereinrichtung 29 gesteuert wird. Das Weichensteuersignal sw wird über die Leitung AK übertragen. Ebenso wird ein Generatorsteuersignal sg über die Leitung AK übertragen. Dazu wird auf Figur 3 verwiesen:

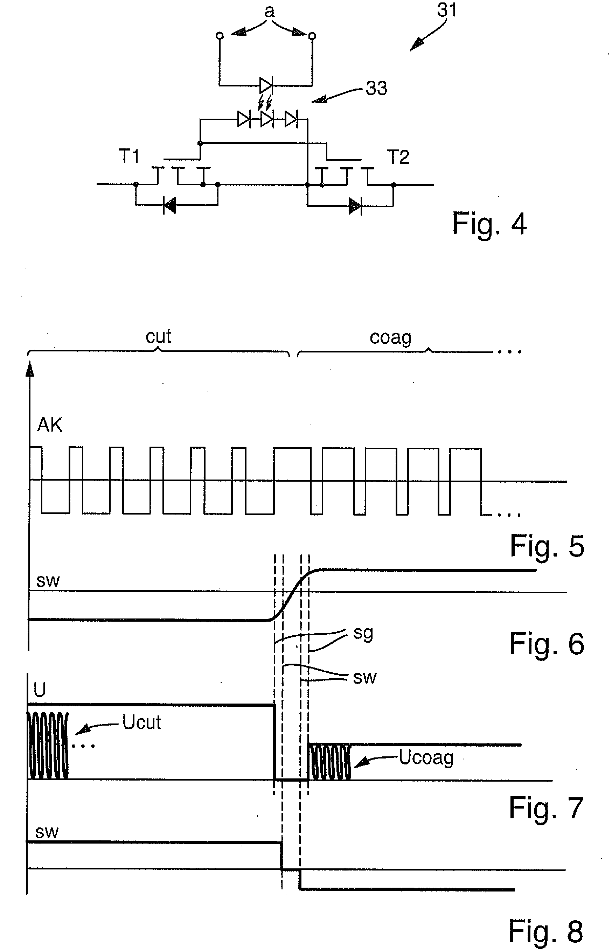

Die Weiche 30 enthält zwei elektronische Schalter 31, 32, die nach dem Vorbild der Figur 4 aufgebaut sein können. Figur 4 veranschaulicht dazu beispielhaft den Schalter 31 mit zwei gegenpolig orientierten, in Reihe geschalteten Feldeffekttransistoren T1, T2, deren Gateelektroden über einen Optokoppler 33 potenzialfrei ansteuerbar sind. Der Optokoppler 33 erhält an seinem Eingang das Signal a, das von der Aktivierungseinrichtung 24 (Figur 3) bereitgestellt wird. Der Schalter 31 kann über einen Koppelkondensator C1 mit der Elektrode 22 verbunden sein. Entsprechend kann der zweite Schalter 32 über einen Koppelkondensator C2 mit der Elektrode 22 verbunden sein.

Optional kann zu der Weiche 30 noch ein dritter Schalter 34 gehören, der den kondensatorseitigen Ausgang des Schalters 31 mit dem Neutralleiter AE verbindet, wenn er das Steuersignal b erhält. Dieser Schalter 34 kann dazu genutzt werden, die Elektrode 20 spannungslos zu halten, während die zum Schneiden dienende Elektrode 22 bestromt ist. In diesem Fall bilden beide Elektroden 20, 21 gemeinsam die Neutralelektrode für den Schneidstrom, der von der Schneidelektrode 22 ausgeht.

Zum Ein- und Umschalten des Generators 28 dienen in oder an der Aktivierungseinrichtung 24 vorgesehene elektrische Schalter 25, 26, beispielsweise in Gestalt von Tastern. Wie in Figur 3 ersichtlich sind diese Schalter 25, 26 mit Widerständen R1, R2 in Reihe zwischen das Neutralpotenzial auf der Leitung AE und die Leitung AK geschaltet. Die Leitung AK führt einen Signalträger. Zur Kommunikation der Aktivierungseinrichtung 24 mit der Steuereinrichtung 29 sowie zur Kommunikation der Steuereinrichtung 29 mit der Weiche 30 sind dem Signalträger das Generatorsteuersignal sg und das Weichensteuersignal sw aufprägbar.

Die Widerstände R1 und R2 können unterschiedliche Werte aufweisen, um den Signalträger der Leitung AK zur Erzeugung des Generatorsteuersignals sg auf unterschiedliche Weise zu beeinflussen. Beispielsweise ist der Signalträger auf der Leitung AK ein rechteckförmiger nullsymmetrischer Wechselstrom mit einer Frequenz von z.B. 2 kHz, mit der er zwischen zwei Stromwerten von +25 mA und -25 mA alterniert. Entsprechend dem Wert des durch Betätigung des Schalters 25 oder 26 aktivierten Widerstands R1 oder R2 erfasst die Steuereinrichtung 29 die sich ergebende Spannung als Generatorsteuersignal sg. Dementsprechend aktiviert sie den Generator 28 entweder in der ersten oder in der zweiten Betriebsart, das heißt zum Beispiel entweder im Koagulationsmodus cut oder im Dissektions- bzw. Schneidmodus cut, woraufhin der Generator 28 die erforderliche Spannung U coag oder Ucut abgibt.

Der Signalträger auf der Leitung AK kann auch ein Spannungssignal sein, das zwischen einem positiven und einem negativen Spannungswert mit einer gegebenen Frequenz, beispielsweise von 2 kHz alterniert. Die Steuereinrichtung 29 erfasst dann den sich einstellenden Strom als Generatorsteuersignal sg und lässt den Generator 28 entsprechend in der signalisierten Betriebsart arbeiten.

Die Steuereinrichtung 29 ist im Weiteren darauf eingerichtet, nach Erfassung eines Generatorsteuersignals sg ein Weichensteuersignal sw zum Steuern der Weiche 30 zu erzeugen und über die Leitung AK auszugeben. Dieses Weichensteuersignal sw wird in dem Instrument 11 von einer Detektoreinrichtung 35 erfasst, die als Mittelwertdetektor 36 ausgebildet sein kann (Figur 3).

Die Steuereinrichtung 29 ist dazu eingerichtet, das Weichensteuersignal sw in dem Tastverhältnis des Signalträgers zu verschlüsseln. Dies ist in Figur 5 exemplarisch veranschaulicht. Das Tastverhältnis wird hier als Verhältnis der Länge einer positiven Spannung oder eines positiven Stroms zur zeitlichen Länge der negativen Spannung oder des negativen Stroms innerhalb einer Welle des in Figur 5 dargestellten Wellenzugs verstanden. Damit ist das Tastverhältnis in dem linken Teil des Diagramms der Figur 5, das mit "cut" gekennzeichnet ist, gering, während es in der rechten, durch die Beschriftung "coag" gekennzeichneten Phase groß ist. Zur Erfassung dieser Verhältnisse dient der Mittelwertdetektor 36, der beispielsweise in Gestalt eines RC-Tiefpasses ausgebildet sein kann. Der Ausgang des Tiefpasses kann mit Leuchtdioden z.B. von Optokopplern verbunden sein, die die Eingänge der jeweiligen elektronischen Schalter 31, 32 (und gegebenenfalls 34) bilden. In Figur 3 sind diese Leuchtdioden schematisch in der Aktivierungseinrichtung 24 veranschaulicht. Sie können jedoch auch in den Schaltern 31, 32, 34 angeordnet sein, wie es Figur 4 nahelegt. Außerdem können zwischen dem Mittelwertdetektor 36 und den Leuchtdioden oder sonstigen Eingängen der Schalter 30, 32, 34 Schalter- oder Verstärkerbauelemente angeordnet sein, deren vorzugsweise hochohmigen Eingänge den Mittelwertdetektor 36, und damit die Spannung auf dem Kondensator C wenig oder nicht belasten.

Die insoweit beschriebene Behandlungseinrichtung 10 arbeitet wie folgt:
Nach Inbetriebnahme ist das Instrument 11, wie in Figur 1 und 2 veranschaulicht, an das Gerät 12 angeschlossen. Soll nun zum Beispiel biologisches Gewebe (z.B. ein Blutgefäß oder sonstiges Gefäß) versiegelt und getrennt werden, wird es mit den Branchen 18, 19 erfasst, die mittels der Betätigungseinrichtung 23 geschlossen werden und das Gewebe zusammendrücken. Durch die Aktivierungseinrichtung 24, insbesondere die Aktivierung zum Beispiel des Schalters 25, wird nun der Generator 28 aktiviert. Dies erfolgt, indem der Schalter 25 geschlossen wird, wodurch Strom durch den Wiederstand R1 und über die Leitung AK fließt. Dies wird von der Steuereinrichtung 29 als Generatorsteuersignal sg erfasst.

Die Generatorsteuereinrichtung 29 interpretiert das Generatorsteuersignal sg als Befehl für die Einnahme des Koagulationsmodus und prägt dem Signalträger auf der Leitung AK die in Figur 5 rechts veranschaulichte Modulation auf. Das Tastverhältnis ist groß, wodurch nach Tiefpassfilterung das positive Weichensteuersignal sw gemäß Figur 6 rechts erfasst und von dem Mittelwertdetektor 26 als Steuersignal a an den Schalter 31 gegeben wird. Der Schalter 31 schließt, d.h. die Weiche 30 verbindet die Elektrode 20 mit der Leitung NE und somit mit dem hochspannungserzeugenden Teil des Generators 28. Die anderen Schalter 32, 34 bleiben offen (nicht leitend). Damit liegt die Koagulationsspannung Ucoag an der Elektrode 20 an, womit der Koagulationsstrom von der Elektrode 20 zu der Elektrode 21 fließen kann.

Soll in den Schneid- oder Dissektionsmodus gewechselt werden, betätigt der Nutzer den anderen Schalter 26. Nunmehr ist der zweite Widerstand R2 zwischen das Neutralpotenzial der Leitung AE und den Signalträger der Leitung AK geschaltet. Dies interpretiert die Steuereinrichtung 29 als Generatorsteuersignal sg für den Schneidmodus. Entsprechend ändert sie die Modulation des Signalträgers (Leitung AK) gemäß Figur 5 links.

Entsprechend dem Generatorsteuersignal sg hat die Generatorspannung U nun, wie Figur 7 zeigt, in dem Schneidmodus einen anderen Wert Ucut (links) als im Koagulationsmodus Ucoag (rechts). Der Mittelwert des Signalträgers ist nun negativ, weswegen der Detektor 35 den Schalter 31 öffnet und die Schalter 32, 34 schließt. Ist dies geschehen, liegt die hochfrequente Spannung U nun, wie Figur 7 links veranschaulicht, mit entsprechend höherer Amplitude als Dissektionsspannung Ucut an dem Ausgang 22 an. Die Dissektionsspannung Ucut wird über die Weiche 30 an die Elektrode 22 geleitet, während der Schalter 34 geschlossen ist. Die beiden Elektroden 20, 21 sind somit parallel geschaltet und bilden gemeinsam die Neutralelektrode für die Schneidelektrode 22.

Wie Figur 8 erkennen lässt, erfolgt das Umschalten der Weiche und das Ein- und Ausschalten des Hochspannungsteils des Generators 28 zeitversetzt. Das Schließen der Schalter 31, 32, 34 erfolgt immer bevor der Hochspannungsteil des Generators 28 aktiviert wird. Umgekehrt wird der Generator 28 immer ausgeschaltet, bevor der zugehörige Schalter der Weiche 30 geöffnet wird. Dies wird aus der in den Figuren 6 und 7 veranschaulichten zeitlichen Abfolge des Generatorsteuersignals sg und des Weichensteuersignals sw ersichtlich.

Der beschriebene Umschaltvorgang kann ein oder mehrmals hintereinander auch in schneller Folge ausgeführt werden, um beispielsweise ein virtuell gleichzeitiges Schneiden und Koagulieren zu erreichen. Es ist sowohl während der ersten Betriebsart, beispielsweise Koagulation, als auch bei der zweiten Betriebsart, beispielsweise Schneiden möglich, Strom und Spannung des Generators oder die von ihm abgegebene Leistung oder den Gewebezustand präzise zu überwachen und den Generator entsprechend zu steuern. Außerdem sind der Strom und die Spannung für jeden Betriebsmodus coag, cut an dem Gerät 12 separat einstellbar.

Neben der oben beschriebenen manuellen Umschaltung zwischen verschiedenen Betriebsarten ist es auch möglich, verschiedene Behandlungsabläufe in dem Generator 28 abzuspeichern und abzurufen. In diesem Fall steuert der Generator 28, nachdem von der Aktivierungseinrichtung 24 ein Startsignal ausgegangen ist, die Weiche 30 entsprechend dem vorgegebenen Behandlungsablauf und gibt je nach Weichenstellung die entsprechenden Spannungen oder Ströme an die jeweils aktivierten Elektroden 20 oder 22 ab.

Die erfindungsgemäße Behandlungseinrichtung 10 umfasst ein an ein speisendes Gerät 12 angeschlossenes Instrument 11. Das Instrument 11 und das Gerät 12 können sich gegenseitig steuern. Mittels einer an dem Instrument 11 oder anderweitig vorgesehenen Aktivierungseinrichtung 24 ist der Generator in einer ersten oder in einer zweiten Betriebsart aktivierbar. Das Instrument 11 enthält eine Weiche 30, um die von dem Generator gelieferte elektrische Leistung auf ein erstes oder ein zweites Elektrodenpaar 20/21 oder 22/21 zu leiten. Die Weiche 30 wird von dem Generator 12 gesteuert. Dies eröffnet die Möglichkeit, Behandlungsabläufe in dem Generator zu speichern und mit dem Instrument 11 auszuführen, ohne dass der Nutzer Betriebsarten umschalten müsste.

### Bezugszeichen:

- 10: Behandlungseinrichtung
- 11: Instrument
- 12: Gerät
- 13: Kabel
- 14: Griff
- 15: Gehäuse
- 16: Schaft
- 17: Werkzeug
- 18: obere Branche
- 19: untere Branche
- 20: obere Elektrode
- 21: untere Elektrode
- 22: Schneidelektrode
- 23: Betätigungseinrichtung
- 24: Aktivierungseinrichtung
- 25, 26: Schalter
- 27: Bedieneinheit
- 28: Generator
- 29: Steuereinrichtung
- NE: Leitung für Behandlungsstrom und Behandlungsspannung
- AE: Leitung für Neutralpotential
- AK: Leitung für den Signalträger
- 30: Weiche
- sw: Weichensteuersignal
- sg: Generatorsteuersignal
- 31, 32: elektronische Schalter
- 33: Optokoppler
- a, b: Steuersignale
- C1, C2: Koppelkondensatoren
- 34: elektronischer Schalter
- 35: Detektoreinrichtung
- 36: Mittelwertdetektor
- R: Widerstand
- C: Kondensator
- U: Spannung
- Ucoag: Koagulationsspannung
- Ucut: Dissektionsspannung

## Patentansprüche

**1.** Behandlungseinrichtung (10), insbesondere zur Koagulation und Dissektion von biologischem Gewebe eines menschlichen oder tierischen Patienten,
mit einem Generator (28), der dazu eingerichtet ist, in Abhängigkeit von einem Generatorsteuersignal (sg) eine elektrische Spannung (U) sowohl mit einem ersten Wert (Ucoag) für einen ersten Behandlungsmodus (coag) als auch alternativ mit einem zweiten Wert (Ucut) für einen zweiten Behandlungsmodus (cut) sowie dementsprechend ein Weichensteuersignal (sw) in einem ersten Zustand oder einem zweiten Zustand abzugeben, der sich von dem ersten Zustand unterscheidet,
mit einem Instrument (11), das wenigstens zwei Elektroden (20, 22) aufweist, die über eine elektrische Leitung (NE) mit dem Generator (28) verbunden sind,
wobei zwischen den Elektroden (20, 22) und der Leitung (NE) eine elektrische Weiche (30) angeordnet ist, die in Abhängigkeit von dem Weichensteuersignal (sw) die eine oder die andere der beiden Elektroden (20, 22) mit der elektrischen Leitung (NE) verbindet, und
mit einer Aktivierungseinrichtung (24), der zur Erzeugung des Generatorsteuersignals (sg) eingerichtet ist.

**2.** Behandlungseinrichtung nach Anspruch 1, **dadurch gekennzeichnet, dass** der Generator (28) ein Hochfrequenzgenerator ist, der darauf eingerichtet ist, eine Spannung mit einer Frequenz zwischen 100 kHz und 5 MHz ist abzugeben.

**3.** Behandlungseinrichtung nach einem der vorstehenden Ansprüche, **dadurch gekennzeichnet, dass** der erste Behandlungsmodus (coag) ein Mode zur Gewebekoagulation ist.

**4.** Behandlungseinrichtung nach einem der vorstehenden Ansprüche, **dadurch gekennzeichnet, dass** der zweite Behandlungsmodus (cut) ein Mode zur Gewebedissektion ist.

**5.** Behandlungseinrichtung nach einem der vorstehenden Ansprüche, **dadurch gekennzeichnet, dass** der erste Wert (Ucoag) der elektrischen Spannung (U) die von positiver Spitze zur negativen Spitze gemessenen Spitzen-Spitzen-Spannung ist, dass der zweite Wert (Ucut) der elektrischen Spannung (U) die von positiver Spitze zur negativen Spitze gemessenen Spitzen-Spitzen-Spannung ist, und dass der erste Wert (Ucoag) geringer ist als der zweite Wert (Ucut) der elektrischen Spannung (U).

**6.** Behandlungseinrichtung nach einem der vorstehenden Ansprüche, **dadurch gekennzeichnet, dass** der Generator (28) eine Steuereinrichtung (29) enthält, die dazu eingerichtet ist, auf einer Leitung (AK) einen Signalträger an die Aktivierungseinrichtung (24) zu senden.

**7.** Behandlungseinrichtung nach Anspruch 6, **dadurch gekennzeichnet, dass** die Aktivierungseinrichtung (24) dazu eingerichtet ist, in Abhängigkeit von einer Nutzereingabe aus dem Signalträger das Generatorsteuersignal (sg) zu generieren.

**8.** Behandlungseinrichtung nach einem der vorstehenden Ansprüche, **dadurch gekennzeichnet, dass** die Aktivierungseinrichtung (24) wenigstens zwei Schalter (25, 26) aufweist, die mit unterschiedlichen elektrischen Widerständen (R1, R2) verbunden sind.

**9.** Behandlungseinrichtung nach einem der vorstehenden Ansprüche, **dadurch gekennzeichnet, dass** die Aktivierungseinrichtung (24) in dem Instrument (11) angeordnet ist.

**10.** Behandlungseinrichtung nach Anspruch 6, **dadurch gekennzeichnet, dass** der Signalträger das Weichensteuersignal (sw) enthält.

**11.** Behandlungseinrichtung nach Anspruch 6, **dadurch gekennzeichnet, dass** der Signalträger ein alternierendes Signal mit variablem Tastverhältnis ist.

**10.** Behandlungseinrichtung nach Anspruch 6, **dadurch gekennzeichnet, dass** das Weichensteuersignal (sw) durch das Tastverhältnis des Signalträgers festgelegt ist.

**11.** Behandlungseinrichtung nach einem der vorstehenden Ansprüche, **dadurch gekennzeichnet, dass** zur Gewinnung des Weichensteuersignals (sw) ein Mittelwertdetektor (36) vorgesehen ist.

**12.** Behandlungseinrichtung nach einem der vorstehenden Ansprüche, **dadurch gekennzeichnet, dass** das Generatorsteuersignal (sg) zeitlich nach dem Weichensteuersignal (sw) beginnend und zeitlich vor dem Weichensteuersignal (sw) endend festgelegt ist.

**15.** Verfahren zur Steuerung einer Behandlungseinrichtung (10) nach Anspruch 1, bei dem:
ein von dem Generator (28) abgegebener Signalträger zu der Aktivierungseinrichtung (24) geleitet wird,
mittels der Aktivierungseinrichtung (24) aus dem Signalträger das dem gewünschten Behandlungsmodus (coag, cut) entsprechende Generatorsteuersignal (sg) erzeugt und dieses dem Generator (28) zugeleitet wird,
der Generator (28) daraufhin das Signalträger verändert, um das Weichensteuersignal (sw) zu erzeugen,
das Weichensteuersignal (sg) in dem Instrument (11) aus dem Signalträger ermittelt und an die Weiche (30) geleitet wird,
wonach die für Weiche (30) die für den gewünschten Behandlungsmodus (coag, cut) geeignete Elektrode (20, 22) mit der Leitung (NE) verbindet,
wonach mittels des Generators (28) die Spannung (Ucoag, Ucut) für den gewünschten Behandlungsmodus (coag, cut) erzeugt und an die Elektrode (20, 22) geleitet wird.

## Geänderte Patentansprüche

### Geänderte Patentansprüche gemäss Regel 137(2) EPÜ.

1. Behandlungseinrichtung (10), insbesondere zur Koagulation und Dissektion von biologischem Gewebe eines menschlichen oder tierischen Patienten,
mit einem Generator (28), der dazu eingerichtet ist, in Abhängigkeit von einem Generatorsteuersignal (sg) eine elektrische Spannung (U) sowohl mit einem ersten Wert (Ucoag) für einen ersten Behandlungsmodus (coag) als auch alternativ mit einem zweiten Wert (Ucut) für einen zweiten Behandlungsmodus (cut) sowie dementsprechend ein Weichensteuersignal (sw) in einem ersten Zustand oder einem zweiten Zustand abzugeben, der sich von dem ersten Zustand unterscheidet,
mit einem Instrument (11), das wenigstens zwei Elektroden (20, 22) aufweist, die über eine elektrische Leitung (NE) mit dem Generator (28) verbunden sind,
wobei zwischen den Elektroden (20, 22) und der Leitung (NE) eine elektrische Weiche (30) angeordnet ist, die in Abhängigkeit von dem Weichensteuersignal (sw) die eine oder die andere der beiden Elektroden (20, 22) mit der elektrischen Leitung (NE) verbindet, und
mit einer Aktivierungseinrichtung (24), der zur Erzeugung des Generatorsteuersignals (sg) eingerichtet ist.

2. Behandlungseinrichtung nach Anspruch 1, **dadurch gekennzeichnet, dass** der Generator (28) ein Hochfrequenzgenerator ist, der darauf eingerichtet ist, eine Spannung mit einer Frequenz zwischen 100 kHz und 5 MHz ist abzugeben.

3. Behandlungseinrichtung nach einem der vorstehenden Ansprüche, **dadurch gekennzeichnet, dass** der erste Behandlungsmodus (coag) ein Mode zur Gewebekoagulation ist.

4. Behandlungseinrichtung nach einem der vorstehenden Ansprüche, **dadurch gekennzeichnet, dass** der zweite Behandlungsmodus (cut) ein Mode zur Gewebedissektion ist.

5. Behandlungseinrichtung nach einem der vorstehenden Ansprüche, **dadurch gekennzeichnet, dass** der erste Wert (Ucoag) der elektrischen Spannung (U) die von positiver Spitze zur negativen Spitze gemessenen Spitzen-Spitzen-Spannung ist, dass der zweite Wert (Ucut) der elektrischen Spannung (U) die von positiver Spitze zur negativen Spitze gemessenen Spitzen-Spitzen-Spannung ist, und dass der erste Wert (Ucoag) geringer ist als der zweite Wert (Ucut) der elektrischen Spannung (U).

6. Behandlungseinrichtung nach einem der vorstehenden Ansprüche, **dadurch gekennzeichnet, dass** der Generator (28) eine Steuereinrichtung (29) enthält, die dazu eingerichtet ist, auf einer Leitung (AK) einen Signalträger an die Aktivierungseinrichtung (24) zu senden.

7. Behandlungseinrichtung nach Anspruch 6, **dadurch gekennzeichnet, dass** die Aktivierungseinrichtung (24) dazu eingerichtet ist, in Abhängigkeit von einer Nutzereingabe aus dem Signalträger das Generatorsteuersignal (sg) zu generieren.

8. Behandlungseinrichtung nach einem der vorstehenden Ansprüche, **dadurch gekennzeichnet, dass** die Aktivierungseinrichtung (24) wenigstens zwei Schalter (25, 26) aufweist, die mit unterschiedlichen elektrischen Widerständen (R1, R2) verbunden sind.

9. Behandlungseinrichtung nach einem der vorstehenden Ansprüche, **dadurch gekennzeichnet, dass** die Aktivierungseinrichtung (24) in dem Instrument (11) angeordnet ist.

10. Behandlungseinrichtung nach Anspruch 6, **dadurch gekennzeichnet, dass** der Signalträger das Weichensteuersignal (sw) enthält.

11. Behandlungseinrichtung nach Anspruch 6, **dadurch gekennzeichnet, dass** der Signalträger ein alternierendes Signal mit variablem Tastverhältnis ist.

12. Behandlungseinrichtung nach Anspruch 6, **dadurch gekennzeichnet, dass** das Weichensteuersignal (sw) durch das Tastverhältnis des Signalträgers festgelegt ist.

13. Behandlungseinrichtung nach einem der vorstehenden Ansprüche, **dadurch gekennzeichnet, dass** zur Gewinnung des Weichensteuersignals (sw) ein Mittelwertdetektor (36) vorgesehen ist.

14. Behandlungseinrichtung nach einem der vorstehenden Ansprüche, **dadurch gekennzeichnet, dass** das Generatorsteuersignal (sg) zeitlich nach dem Weichensteuersignal (sw) beginnend und zeitlich vor dem Weichensteuersignal (sw) endend festgelegt ist.

15. Verfahren zur Steuerung einer Behandlungseinrichtung (10) nach Anspruch 1, bei dem:
ein von dem Generator (28) abgegebener Signalträger zu der Aktivierungseinrichtung (24) geleitet wird,
mittels der Aktivierungseinrichtung (24) aus dem Signalträger das dem gewünschten Behandlungsmodus (coag, cut) entsprechende Generatorsteuersignal (sg) erzeugt und dieses dem Generator (28) zugeleitet wird,
der Generator (28) daraufhin das Signalträger verändert, um das Weichensteuersignal (sw) zu erzeugen,
das Weichensteuersignal (sg) in dem Instrument (11) aus dem Signalträger ermittelt und an die Weiche (30) geleitet wird,
wonach die für Weiche (30) die für den gewünschten Behandlungsmodus (coag, cut) geeignete Elektrode (20, 22) mit der Leitung (NE) verbindet,
wonach mittels des Generators (28) die Spannung (Ucoag, Ucut) für den gewünschten Behandlungsmodus (coag, cut) erzeugt und an die Elektrode (20, 22) geleitet wird.
